# EUROPEAN PATENT SPECIFICATION

(11) **EP 3 458 592 B1**
(45) Date of publication and mention of the grant of the patent: **11.03.2026**
(21) Application number: 17800238.2
(22) Date of filing: 19.05.2017
(51) Int. Cl.: A01N 35/02, A01N 37/06, A01N 43/12, A01P 21/00

(54) **USE OF PLANT GROWTH REGULATORS**
VERWENDUNG VON PFLANZENWACHSTUMSREGULATOREN
UTILISATION DE RÉGULATEURS DE CROISSANCE DES PLANTES

(30) Priority: 19.05.2016 US 201662338590 P
(43) Date of publication of application: 27.03.2019
(73) Proprietor: Duke University, Durham, NC 27705 (US)
(72) Inventor: BENFEY, Philip, Durham NC 27705 (US); DICKINSON, Jazz, Durham NC 27705 (US)
(74) Representative: Harris, Oliver John Richard
(86) International application number: PCT/US2017/033547
(87) International publication number: WO 2017/201410

(56) References cited:
- WO-A2-00/49865
- US-A1- 2008 227 645
- US-A1- 2008 227 645
- US-A1- 2011 230 352
- US-A1- 2015 299 061
- HINANIT KOLTAI: "Strigolactones are regulators of root development", NEW PHYTOLOGIST, vol. 190, no. 3, 9 March 2011 (2011-03-09), GB, pages 545 - 549, XP055440302, ISSN: 0028-646X, DOI: 10.1111/j.1469-8137.2011.03678.x
- KALSI P S ET AL: "Conjugated terpenoid ketones: A new group of plant growth regulators", EXPERIENTA,, vol. 35, no. 4, 1 April 1979 (1979-04-01), pages 481 - 482, XP001464969
- S D SALT ET AL: "Effects of beta-ionone and abscisic acid on the growth of tobacco and resistance to blue mold. Mimicry of effects of stem infection by Peronospora tabacina Adam", 1 January 1986 (1986-01-01), pages 287 - 297, XP055634868, Retrieved from the Internet <URL:https://www.sciencedirect.com/science/article/pii/S0048405986800716/pdf?md5=96842d3e0e5eee19f87a96e48fc493f7&pid=1-s2.0-S0048405986800716-main.pdf> [retrieved on 20191022]
- F. RAMEL ET AL: "Carotenoid oxidation products are stress signals that mediate gene responses to singlet oxygen in plants", PROCEEDINGS OF THE NATIONAL ACADEMY OF SCIENCES, vol. 109, no. 14, 19 March 2012 (2012-03-19), US, pages 5535 - 5540, XP055662331, ISSN: 0027-8424, DOI: 10.1073/pnas.1115982109
- FANNY RAMEL ET AL: "Chemical Quenching of Singlet Oxygen by Carotenoids in Plants", PLANT PHYSIOLOGY, vol. 158, no. 3, 10 January 2012 (2012-01-10), Rockville, Md, USA, pages 1267 - 1278, XP055662352, ISSN: 0032-0889, DOI: 10.1104/pp.111.182394
- FEIFEI LV ET AL: "[beta]-cyclocitral upregulates salicylic acid signalling to enhance excess light acclimation in Arabidopsis", JOURNAL OF EXPERIMENTAL BOTANY, vol. 66, no. 15, 21 May 2015 (2015-05-21), GB, pages 4719 - 4732, XP055662353, ISSN: 0022-0957, DOI: 10.1093/jxb/erv231
- FRIEDRICH J�TTNER ET AL: "Beta-Cyclocitral, a Grazer Defence Signal Unique to the Cyanobacterium", JOURNAL OF CHEMICAL ECOLOGY, KLUWER ACADEMIC PUBLISHERS-PLENUM PUBLISHERS, NE, vol. 36, no. 12, 12 November 2010 (2010-11-12), pages 1387 - 1397, XP019855112, ISSN: 1573-1561, DOI: 10.1007/S10886-010-9877-0
- TOMITA ET AL, ENVIRON. SCI. POLLUT. RES,, vol. 23, 1 January 2016 (2016-01-01), pages 11998 - 12006, XP002769587
- T. MITSUI ET AL: "The growth promoting activity for plants of the substances which have carboxyl group(s) attached directly to the ring system. Studies on the plant hormones VI", NIPPON NOGEI KAGAKU KAISHI, 1 January 1952 (1952-01-01), pages 63 - 66, XP055367437
- KOLTAI: "Strigolactones are regulators of root development", NEW PHYTOLOGIST, vol. 190, 2011, pages 545 - 549, XP055440302
- EROGLU ET AL.: "Carotenoid Metabolism in Mammals, Including Man: Formation, Occurrence, and Function of Apocarotenoids", JOURNAL OF LIPID RESEARCH, vol. 54, 2013, pages 1719 - 1730, XP055440307
- SAHA, SUPPLYING PLANT NUTRIENTS VIA FERTIGATION: PRINCIPLES AND EXMAPLES IN TRIPLOID WATERMELON, 2012, pages 3 - 4, XP055440309, Retrieved from the Internet <URL:https://ag.purdue.edu/h!a/fruitveg/Presentations/Saha_Fertigation_6up.pdf>

## Description

### CROSS-REFERENCE TO RELATED APPLICATIONS

### STATEMENT REGARDING FEDERALLY SPONSORED RESEARCH OR DEVELOPMENT

This invention was made with United States government support awarded by NIH Grant No. R01-GM043778. The United States government has certain rights in this invention.

### BACKGROUND OF THE INVENTION

### Field of the Invention

This disclosure generally relates to methods for regulating growth in a plant, for example by administration of a composition comprising an effective amount of one or more apocarotenoids to the plant, its part, or the seed.

### Description of the Related Art

The rapidly increasing world population, coinciding with changes in climate, creates a need for new ways to stabilize and improve plant growth under harsh environmental conditions. One such way is to regulate root branching and root growth, which are essential for nutrient and water uptake. Two promising methods of optimizing root architecture for increased plant performance are to regulate root system depth and branching (Rogers, E.D. and P.N. Benfey, Current Opinion in Biotechnology, 2015. 32: p. 93-98; Manschadi, A.M., et al., Functional Plant Biology, 2006. 33(9): p. 823-837; Champoux, M.C., et al., Theoretical and Applied Genetics, 1995. 90(7-8): p. 969-981). In developing *Arabidopsis* plants, branching is initiated by the formation of *de novo* lateral root primordia. Positioning of lateral root primordia is controlled by a "lateral root clock" - an oscillation cycle of gene expression changes that establishes sites competent to form primordia (Moreno-Risueno et al., Science, 2010. 329 (5997): p. 1306-1311).

The carotenoid pathway is a rich source of metabolites, called apocarotenoids, several of which are important regulators of plant development and root system architecture. Strigolactones, hormones derived from β-carotene, reduce root growth and lateral root and shoot branching (Gomez-Roldan, V., et al., Nature, 2008. 455(7210): p. 189-U22; Ruyter-Spira, C., et al., Plant Physiology, 2011. 155(2): p. 721-734). Abscisic acid (ABA), a stress-responsive hormone metabolite of zeaxanthin, has many roles throughout plant development, including in germination, abscission, drought response and root growth (De Smet, I., et al., Trends in Plant Science, 2006. 11(9): p. 434-439). Previously, an inhibitor of carotenoid metabolism, called D15, was found to decrease lateral root branching through an ABA and strigolactone independent-mechanism in *Arabidopsis thaliana* (Van Norman, J.M., et al., Proceedings of the National Academy of Sciences of the United States of America, 2014. 111(13): p. E1300-E1309.) Note that US2008/227645A1 discloses use of abscisic acid on ornamental plants, Koltai (New Phytologist, vol. 190, no. 3, 09 March 2011, pages 545-549) discloses that strigolactones are regulators of root development, Kalsi et al (Experimenta, vol. 35, no. 4, 01 April 1979, pages 481-482) discloses conjugated terpenoid ketones as plant growth regulators, WO00/49865A2 discloses the use of various terpenes against various organisms, Salt et al (Physiological and Molecular Plant Pathology, vol. 28, no. 2, 01 January 1986, pages 287-297) discloses the effects of beta-ionone and abscisic acid on the growth and resistance to blue mold, Ramel et al (Proc. of the National Academy of Sciences, vol. 109, no. 14, 19 March 2012, pages 5535-5540) discloses that carotenoid oxidation products are stress signals that mediate gene responses to singlet oxygen in plants, Ramel et al (Plant Physiology, vol. 158, no. 3, 10 January 2012, pages 1267-1278) discloses chemical quenching of singlet oxygen by carotenoids in plants, Lv et al (Journal of Experimental Botany, vol. 66, no. 15, 21 May 2015, pages 4719-4732) discloses that beta-cyclocitral upregulates salicyclic acid signalling to enhance excess light acclimation in Arabidopsis, Juttner et al (J. Chem. Ecology, vol. 36, no. 12, 12 November 2010, pages 1387-1397) discloses that beta-cyclocitral is a grazer defence signal unique to the cyanobacterium Microcystis, Tomita et al (Environ. Sci. Pollut. Res., vol. 23, 01 January 2016, pages 11998-12006) discloses oxidation behaviour of beta-cyclocitral from Microcystis, and that Mitsui et al (Nippon Nogei Kagaku Kaishi, 01 January 1952, pages 63-66) discloses the growth promoting activity for plants of the substances which have carboxyl group(s) attached directly to the ring system.

### SUMMARY OF THE INVENTION

Natural signaling molecules capable of increasing root depth and branching are highly desirable tools for enhancing stress tolerance in crops. The inventors have found novel regulators of root architecture in plants when applied exogenously to the plants.

The claimed invention provides a method for improving growth in a plant, the method comprising exogenously contacting a composition comprising an effective amount of β-cyclocitral to the plant, a plant part, or a plant seed, wherein the improving growth comprises improving one or more of primary root growth, lateral root growth, crown root growth, and lateral root branching.

The claimed invention also provides a method for improving drought tolerance in a plant, the method comprising exogenously contacting a composition comprising an effective amount of β-cyclocitral to the plant, a plant part, or a plant seed. The claimed invention also provides a method for reducing growth in a plant, the method comprising exogenously contacting a composition comprising an effective amount of β-cyclocitral, safranal, dimethyl-β-cyclocitral, or pseudoionone, or a combination thereof, to the plant, a plant part, or a plant seed.

Described, but not according to the claimed invention, are methods for fertilizing plant soil, the method comprising providing a composition comprising an effective amount of one or more apocarotenoids to the soil.

Described, but not according to the claimed invention, are compositions comprising, consisting of, or consisting essentially of one or more an effective amount of one or more apocarotenoids present in the composition in a concentration of about 0.01 µM to about 100 mM and an agriculturally acceptable carrier, excipient, and/or diluent.

### BRIEF DESCRIPTION OF THE DRAWINGS

The accompanying drawings are included to provide a further understanding of the methods and compositions of the disclosure, and are incorporated in and constitute a part of this specification. The drawings illustrate one or more embodiment(s) of the disclosure, and together with the description serve to explain the principles and operation of the disclosure, noting that only β-cyclocitral, safranal, dimethyl-β-cyclocitral, and pseudoionone are as such according to one or more aspects of the claimed invention, as set out in the appended claims.

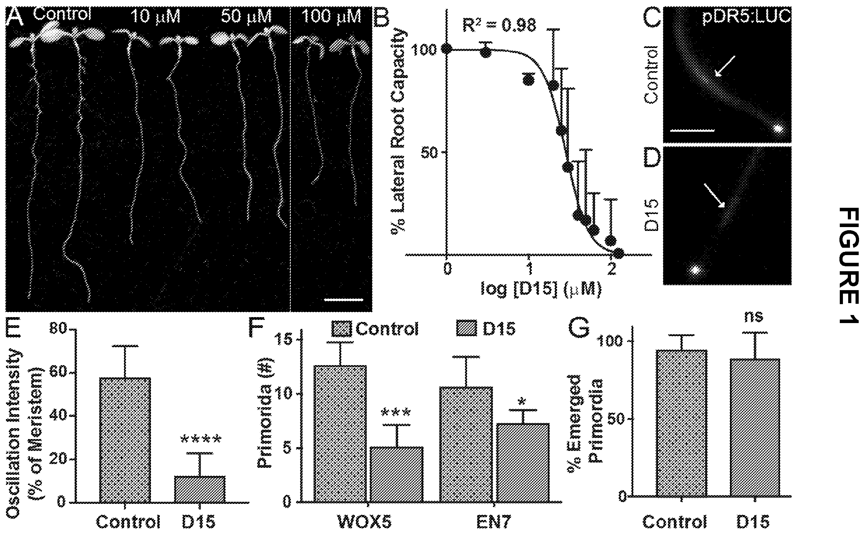

**Figure 1** shows that D15 (N-(4-fluorobenzyl)-methoxycinnamic hydroxamic acid) prevents branching by inhibiting the initiation of lateral root primordia. A) Plants treated with varying concentrations of D15. Scale bar = 5 mm. B) The lateral root capacity (defined as the number of emerged lateral roots after removal of the primary root apical meristem) of D15-treated plants, normalized to control plants. C-D) Luminescence images of the root clock in primary roots during the peak of oscillation intensity in a control root (C) and a root treated with 100 µM D15 (D) Scale bar = 0.5 mm. The arrows indicate the center of the oscillation zone. E) The average oscillation zone intensity during the peak of oscillation in control and D15-treated roots. F) The number of primordia identified using WOX5 and EN7 markers for stage 1 and stage 3 primordia, respectively. G) The percent of primordia that emerged into fully developed lateral roots in control and D15-treated plants. For all graphs, symbols *, **, ***, and **** indicate p values ≤0.05, 0.01, 0.001, and 0.0001, respectively.

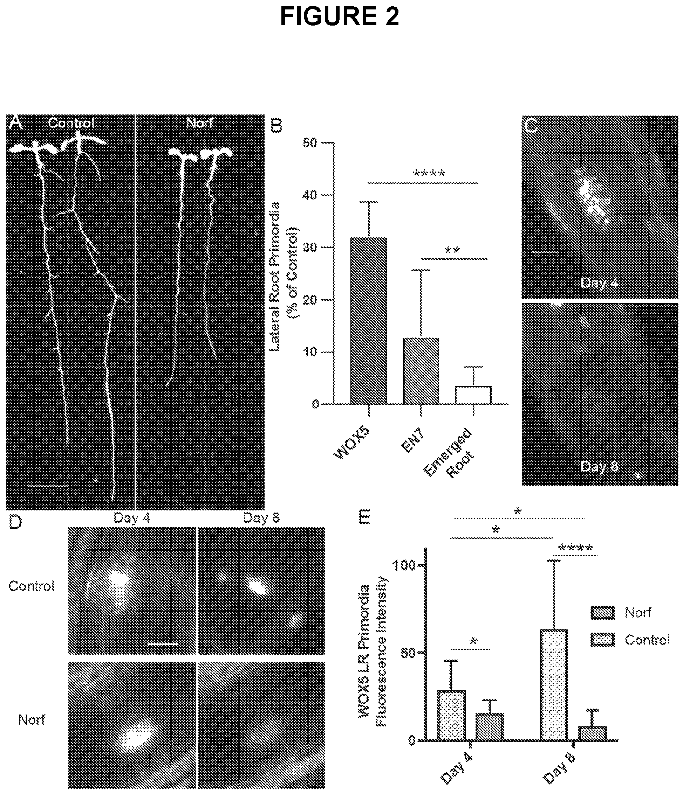

**Figure 2** shows that norflurazone (Norf) (4-chloro-5-(methylamino)-2-(α,α,α-trifluoro-m-tolyl)-3(2H)-pyridazinone), a carotenoid biosynthesis inhibitor, blocks normal lateral root development. A) Control (left) and Norf-treated plants (right). B) The number of lateral root primordia identified either by WOX5 (stage 1 marker), EN7 (stage 3 marker), or by full emergence, compared to control roots. C) Lateral root primordium in pEN7:GAL4; UAS:H2A-GFP plants treated with Norf. At day 4, the endodermis is clearly marked in the developing primordium. By day 8, EN7 is no longer visible, indicating that this primordium has become dormant. D-E) WOX5 positive lateral root primordia increase in brightness in control-treated roots, but decrease in Norf-treated roots, again suggesting that Norf causes primordia to become dormant.

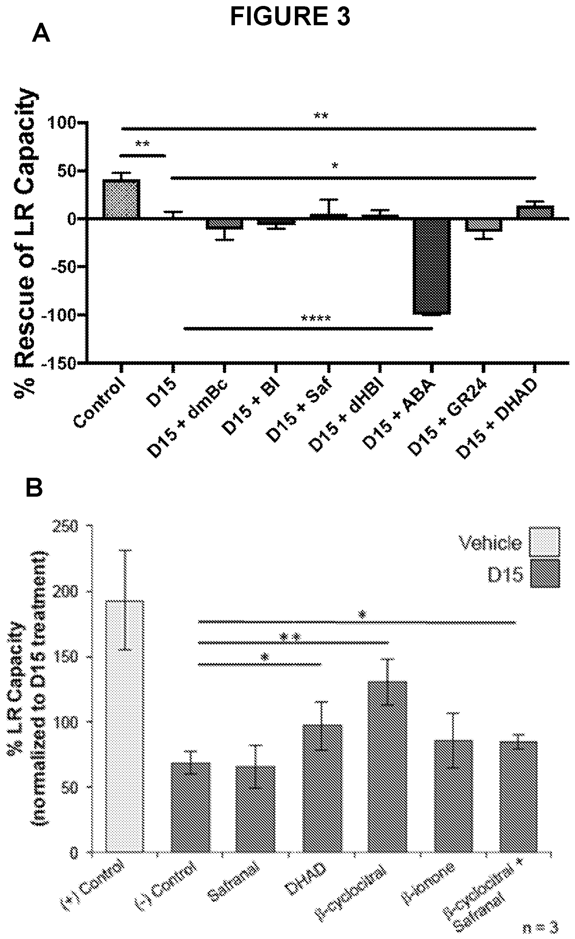

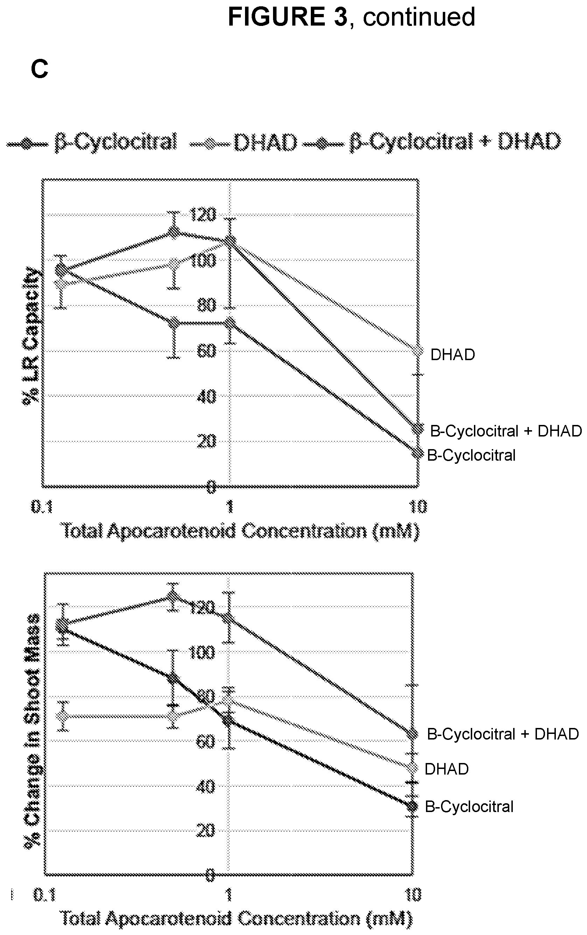

**Figure 3** illustrates the effect of certain apocarotenoids on rescuing the lateral root (LR) capacity of D15-treated plants (normalized to D15 treatment). A) Abbreviations: dimethyl-β-cyclocitral (dmBc), β-ionone (BI), safranal (Saf), abscisic acid (ABA), dihydroactinidiolide (DHAD), strigolactone (GR24), and dihydro-β-ionone (dHBI). B) The D15-treated plants were sprayed daily with a total concentration of 125 µM of the indicated apocarotenoid(s). C) The plants not treated with D15 were sprayed daily with a varying concentration of apocarotenoid(s). The top graph shows the percent change in lateral root capacity and the bottom graph shows the percent change in shoot mass (both results normalized to control plants).

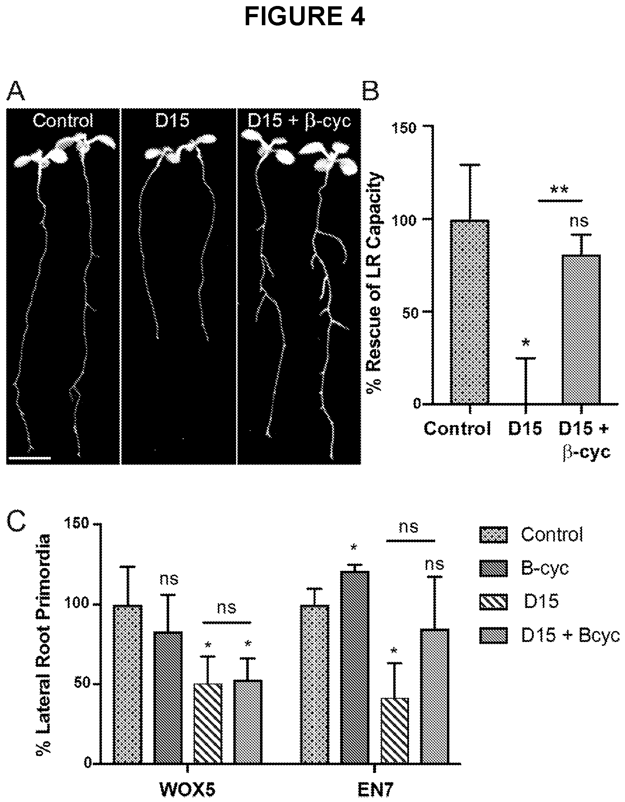

**Figure 4** illustrates the effect of β-cyclocitral on lateral root branching and growth in *Arabidopsis.* A-B) β-cyclocitral can fully rescue lateral root branching when applied to D15-treated plants. C) β-cyclocitral does not increase the number of WOX5⁺ primordia, but does significantly increase the number of EN7⁺ primordia.

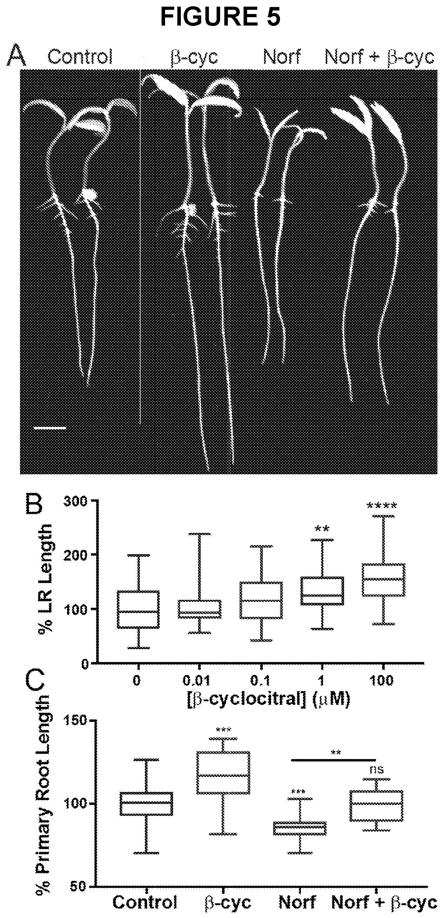

**Figure 5** illustrates the role of the carotenoid pathway in primary and lateral root growth in tomatoes. A) Plants treated with a vehicle control, β-cyclocitral (β-cyc), norflurazone (Norf), and a combination of norflurazone plus β-cyclocitral. Scale bar = 1 cm. B) The effect of changes in β-cyclocitral concentration on lateral root length, normalized to the control treatment. C) Changes in primary root length as a response to β-cyclocitral and norflurazone treatments, normalized to the control treatment.

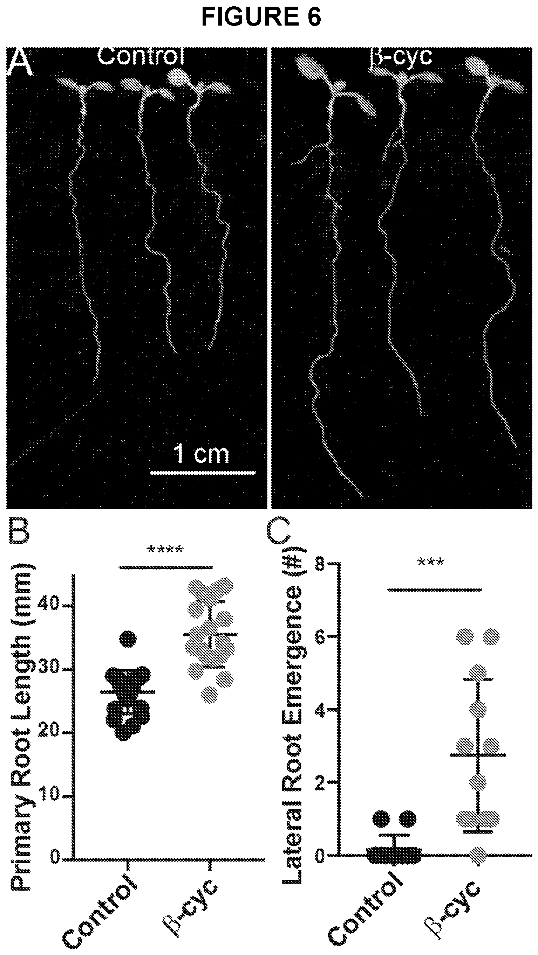

**Figure 6** illustrates the role of β-cyclocitral on primary root length and lateral root emergence in Arabidopsis in the absence of D15. A) Comparing root growth in control and β-cyclocitral treated plants. B-C) Quantification of β-cyclocitral's effect on primary root length (B) and unstimulated (natural) lateral root emergence (C).

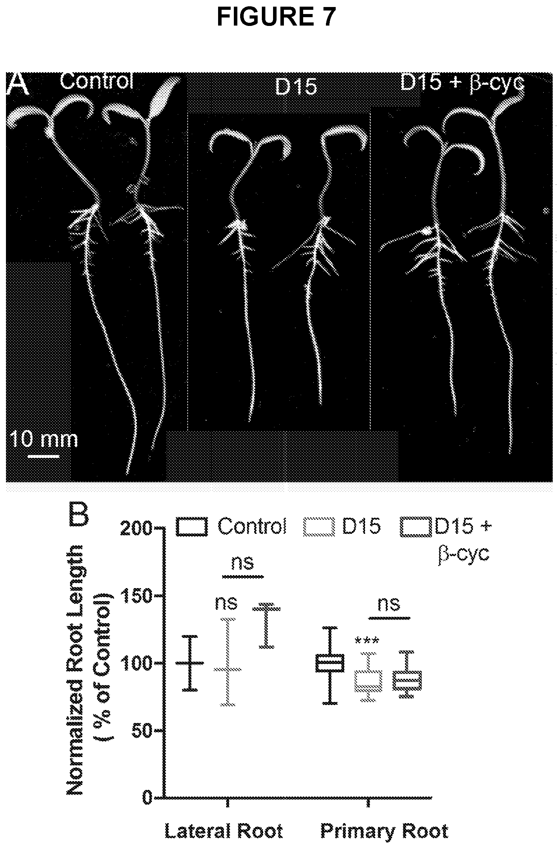

**Figure 7** illustrates the role of D15 in primary and lateral root growth in tomatoes. A-B) D15 decreases primary root length in Heinz tomatoes, but does not decrease lateral root length. D15 inhibition of primary roots is not rescued by β-cyclocitral.

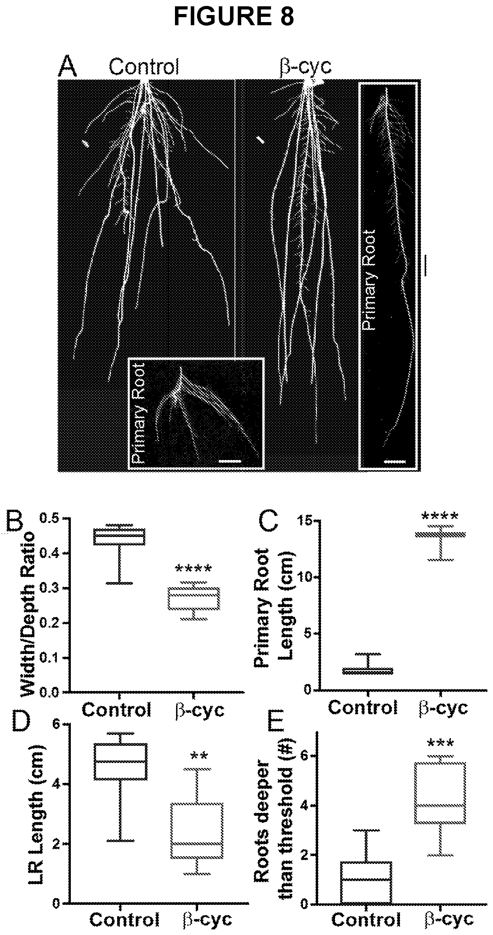

**Figure 8** illustrates the role of β-cyclocitral on rice root systems. A) Representative images of *Azucena* roots treated with a vehicle control and 10 µM β-cyclocitral. The insets show the dissected primary roots in both treatments. Scale bar = 5 mm. B-D) Quantification of the effect of β-cyclocitral on root system architecture traits: ratio of the total width to the total depth of the root system (B), length of the primary root (C), length of the longest lateral root (D), and the total number of roots per plant (including the primary root and crown roots, if applicable) that grew deeper than 12 cm (E).

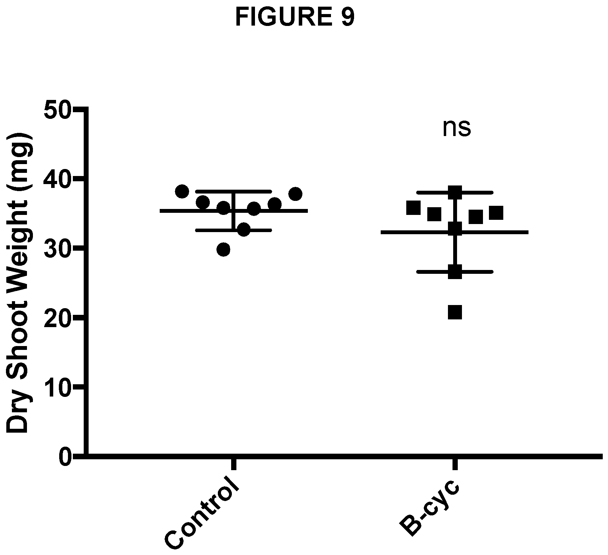

**Figure 9** shows that β-cyclocitral does not significantly change the dry shoot weight of rice plants.

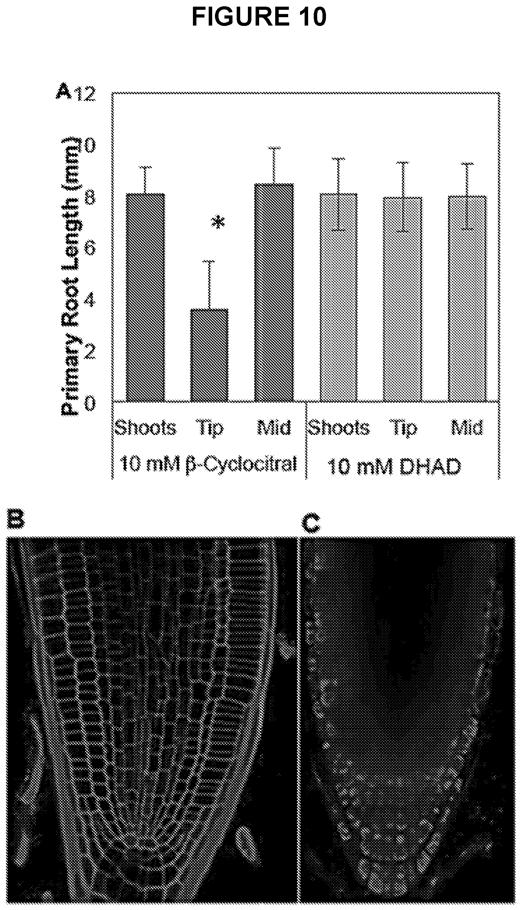

**Figure 10** illustrates the effect of high doses of β-cyclocitral and DHAD on root growth. A) The effect of adding small volumes of 10 mM apocarotenoids daily to either the shoots, root tip, or the middle of the root. B) A confocal image of a healthy root stained with propidium iodide, which marks dead cells. C) A root treated with 10 mM β-cyclocitral applied to the tip of the root.

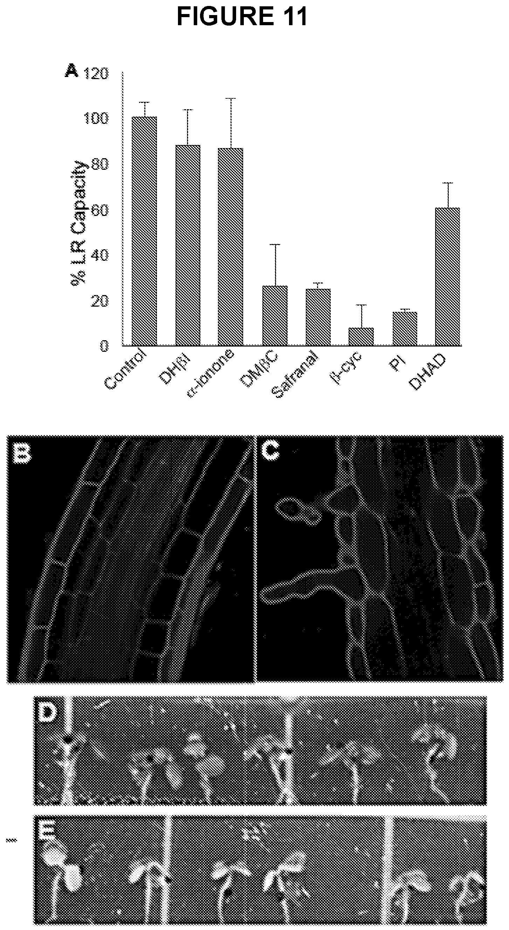

**Figure 11** illustrates the effect of 10 mM apocarotenoids on plant development. A) The percent change in lateral root capacity, normalized to control plants. Abbreviations: dihydro-β-ionone (DHβI), dimethyl-β-cyclocitral (DMβC), β-cyclocitral (β-cyc), pseudoionone (PI), and dihydroactinidiolide (DHAD). B) Confocal image of a control root stained with propidium iodide. C) Image of a root treated with safranal. D) Images of leaves form a control plant. E) Images of leaves from a plant treated with pseudoionone.

### DETAILED DESCRIPTION OF THE INVENTION

Before the disclosed processes and materials are described, it is to be understood that the aspects described herein are not limited to specific embodiments, apparati, or configurations, and as such can, of course, vary. It is also to be understood that the terminology used herein is for the purpose of describing particular aspects only and, unless specifically defined herein, is not intended to be limiting.

Throughout this specification, unless the context requires otherwise, the word "comprise" and "include" and variations (e.g., "comprises," "comprising," "includes," "including") will be understood to imply the inclusion of a stated component, feature, element, or step or group of components, features, elements or steps but not the exclusion of any other integer or step or group of integers or steps.

As used in the specification and the appended claims, the singular forms "a," "an" and "the" include plural referents unless the context clearly dictates otherwise.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

As used herein, the term "contacting" includes the physical contact of at least one substance to another substance.

As used herein, the term "root architecture" means the spatial arrangement of a plant's root tissue within the soil.

As used herein, the term "improving growth" refers to promoting, increasing or improving the rate of growth of the plant or increasing or promoting an increase in the size of the plant.

As used herein, the term "reducing growth" refers to decreasing or slowing the rate of growth of the plant or decreasing or promoting a decrease in the size of the plant.

All percentages, ratios and proportions herein are by weight, unless otherwise specified. A weight percent (weight %, also as wt %) of a component, unless specifically stated to the contrary, is based on the total weight of the formulation or composition in which the component is included (e.g., on the total amount of the catalyst material).

In view of the present disclosure, the methods and compositions described herein can be configured by the person of ordinary skill in the art to meet the desired need. In general, the disclosed materials, methods, and apparati relate to apocarotenoids that can modify root architecture when applied exogenously to plants. Root architecture plays a major role in plant fitness, crop performance, and/or grain yield. The inventors have found novel regulators of root architecture in plants when applied exogenously to the plants. For example, nanomolar concentrations of apocarotenoids of the disclosure (e.g., β-cyclocitral) were found to fully rescue lateral root branching in the presence of an apocarotenoid synthesis inhibitor. In addition, apocarotenoids of the disclosure (e.g., β-cyclocitral) increased primary and lateral root growth, even in the absence of inhibitors. Lateral roots constitute the majority of a mature plant's root system and are vital for plant anchorage, absorption of water and nutrients, and survival in the presence of diverse environmental stresses such as drought. One of the apocarotenoids of the disclosure, β-cyclocitral, was also found to significantly increase primary root growth, as well as lateral and crown root growth, in tomatoes and rice, respectively.

Described herein are methods for regulating growth in a plant, the method comprising exogenously contacting a composition comprising an effective amount of one or more apocarotenoids to the plant, a plant part, or a plant seed.

The plants may be regulated to improve growth of the plant (as per one aspect of the claimed invention). For example, the growth may be improved by modifying the root architecture of the plant. The growth also may be improved by altering (e.g., improving) lateral root formation in the plant. The lateral roots of a plant, which constitute the majority of a mature plant's root system, are vital for plant anchorage, absorption of water and nutrients, and survival in the presence of diverse environmental stresses such as drought. Optionally, the growth of the plant is improved by at least about 10% compared to the plant not contacted with the composition of the disclosure. For example, the growth of the plant can be improved by at least about 12%, or by at least about 15%, by at least about 17%, or by at least about 20%, or by at least about 25%, or by at least about 30%, or by at least about 40%, or by at least about 50%, or by at least about 75%, or even by at least 100% or more as compared to the plant not contacted with the composition.

The plants may be regulated to reduce growth of the plant (as per one aspect of the claimed invention). This method may be particularly useful in controlling unwanted plants, such as weeds, that grow among plant crops (i.e., act as herbicides). For example, the growth may be reduced by modifying the root architecture of the plant. Optionally, the growth of the plant is reduced by at least about 20% compared to the plant not contacted with the composition of the disclosure. For example, the growth of the plant can be reduced by at least about 25%, or by at least about 30%, or by at least about 40%, or by at least about 50%, or by at least about 75%, or even by at least 100% or more as compared to the plant not contacted with the composition.

Also described are methods for improving drought tolerance in a plant, the method comprising exogenously contacting a composition comprising an effective amount of one or more apocarotenoids to the plant, a plant part, or a plant seed (with a specific version thereof according to another aspect of the claimed invention). The improvement of drought tolerance is essential for stable and adequate crop production in drought-prone areas. Recent studies have determined that alteration of root system architecture affects (e.g., improves) drought tolerance (Uga et al., Nature Genetics, 2013, 45:1097-1102; Comas et al., Front. Plant Sci., 2013, 4:442; Rogers and Benfey, Curr Opin Biotechnol. 2015, 32:93-8; Manschadi et al., Functional Plant Biology, 2006, 33:823-837). The composition comprising an effective amount of one or more apocarotenoids, such as β-cyclocitral, improves plant's root systems by, for example, making them deeper and more compact, which enhances drought tolerance.

Also described (but not according to the claimed invention) are methods for fertilizing plant soil, the method comprising providing a composition comprising an effective amount of one or more apocarotenoids to the soil.

The methods of the disclosure require a composition comprising, consisting of, or consisting essentially of one or more apocarotenoids. As used herein, the term "apocarotenoid" means a cleavage product derived from one or more carotenoids. Most apocarotenoids are normally present at very low levels in the roots of plants. Apocarotenoids are formed by the action of nine different carotenoid cleavage dioxygenases (CCDs). The specific products generated by each individual CCD are difficult to characterize because CCDs seem to have functional redundancy. For instance, although it is known that D15 (N-(4-fluorobenzyl)-methoxycinnamic hydroxamic acid) can inhibit CCD8, *ccd8* mutants do not have reduced lateral root capacity. In fact, even quadruple *ccd8*/*ccd1*/*ccd4*/*ccd7* mutants (which are the most closely related of the CCDs), do not have a lateral root phenotype, suggesting that CCDs are highly functionally redundant. This makes it very difficult to specifically reduce the levels of a particular apocarotenoid, such as β-cyclocitral, using genetic approaches. In addition, overexpression or mutagenesis of one or more CCDs that normally produce a particular apocarotenoid would likely affect other, less desired, apocarotenoids. This makes it extremely challenging to change endogenous levels of a particular apocarotenoid in plants using genetic approaches. The inventors have found that exogenous application of a composition comprising one or more apocarotenoids can profoundly alter root architecture.

The apocarotenoids contemplated by the disclosure are β-ionone, β-cyclocitral, safranal, dihydro-β-lonone, dimethyl-β-cyclocitral, dihydroactinidiolide (DHAD), α-ionone, and pseudoionone, and combinations thereof. The chemical structures of these compounds are provided below:

| | |
|---|---|
| 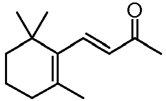 β-ionone | 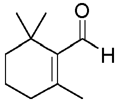 β-cyclocitral |
| 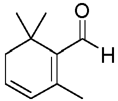 safranal | 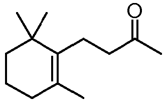 dihydro-β-ionone |
| 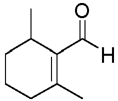 dimethyl-β-cyclocitral | 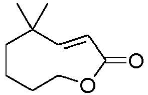 dihydroactinidiolide (DHAD) |
| 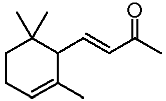 α-ionone | 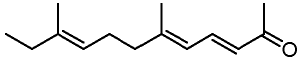 Pseudoionone |
| 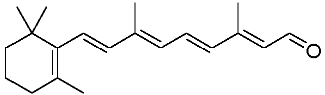 Retinal | 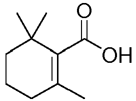 2,6,6-trimethyl-1-cyclohexene-1-carboxylic acid |
| 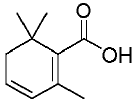 2,6,6-trimethylcyclohexa-1,3-diene-1-carboxylic acid | 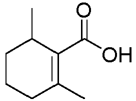 2,6-dimethylcyclohex-1-ene-1-carboxylic acid |

The apocarotenoid of the disclosure can be selected from the group consisting of β-ionone, β-cyclocitral, safranal, dihydro-β-lonone, dimethyl-β-cyclocitral, dihydroactinidiolide (DHAD), α-ionone, pseudoionone, and combinations thereof.

The apocarotenoid of the disclosure can be β-cyclocitral. β-cyclocitral is particularly useful in methods to improve growth of the plant.

The apocarotenoid of the disclosure can be selected from the group consisting α-ionone, β-ionone, safranal, dimethyl-β-cyclocitral, pseudoionone, or a combination thereof.

In certain embodiments, the apocarotenoid of the disclosure is present in the composition in a concentration of about 0.01 µM to about 100 mM. For example, in certain embodiments, the apocarotenoid of the disclosure is present in the composition in a concentration of about 0.01 µM to about 50 mM, or about 0.01 µM to about 50 mM, or about 0.01 µM to about 10 mM, or about 0.01 µM to about 5 mM, or about 0.01 µM to about 2 mM, or about 0.01 µM to about 1 mM, or about 0.01 µM to about 500 µM, or about 0.1 µM to about 100 mM, or about 0.1 µM to about 50 mM, or about 0.1 µM to about 50 mM, or about 0.1 µM to about 10 mM, or about 0.1 µM to about 5 mM, or about 0.1 µM to about 2 mM, or about 0.1 µM to about 1 mM, or about 0.1 µM to about 500 µM, or about 1 µM to about 100 mM, or about 1 µM to about 50 mM, or about 1 µM to about 50 mM, or about 1 µM to about 10 mM, or about 1 µM to about 5 mM, or about 1 µM to about 2 mM, or about 1 µM to about 1 mM, or about 1 µM to about 500 µM. Moreover, the apocarotenoid of the disclosure can be present in the composition in a concentration of any of the minimums and maximums provided above.

In certain embodiments, the apocarotenoid of the disclosure is present in the composition in a concentration of about 0.01 µM to about 1 mM, or about 0.01 µM to about 500 µM, or about 0.1 µM to about 100 µM, or about 0.05 µM to 0.15 µM, or about 50 µM to about 150 µM, or about 5 µM to about 15 µM. Such concentrations can be useful for improving plant growth. The concentration of about 0.05 µM to 0.15 µM can be useful in improving the growth of *Arabidopsis.* The concentration of about 50 µM to 150 µM can be useful in improving the growth of tomato. The concentration of about 5 µM to 15 µM can be useful in improving the growth of rice.

In certain embodiments, the apocarotenoid of the disclosure is present in the composition in a concentration of more than about 1 mM, or more than about 10 mM, or more than about 20 mM, or about 1 mM to about 10 mM, or about 1 mM to about 20 mM, or about 1 mM to about 50 mM, or about 5 mM to about 10 mM, or about 5 mM to about 15 mM, or about 5 mM to about 20 mM, or about 10 mM to about 100 mM, or about 10 mM to about 20 mM. Such concentrations can be useful for reducing plant growth.

The compositions of the disclosure may further comprise one or more substances formulated for at least one agricultural application. For example, the compositions of the disclosure can further comprise one or more of agriculturally acceptable carrier, excipient, and/or diluent. Examples include, but are not limited to fertilizers (e.g., calcium, nitrogen, potassium, phosphorous), micronutrients (e.g., metal ions), insecticides, fungicides, nematocides, bactericides, acaricides, herbicides, plant nutrients, rooting stimulants, chemosterilants, semiochemicals, repellents, attractants, pheromones, feeding stimulants, microbial inocula or entomopathogenic bacteria, viruses, fungi, and other signal compounds including apocarotenoids, flavonoids, jasmonates and strigolactones (Akiyama et al., Nature, 2005, 435:824-827; Harrison, Ann. Rev. Microbiol., 2005, 59:19-42; Besserer et al., PLoS Biol., 2006, 4(7):e226; International Patent Publication no. WO 2009/049747). These compounds can also be formulated into mixtures or multi-component formulations. Agricultural applications are understood to include, but not be limited to, yield improvement, pest control, disease control, weed control, and resistance to abiotic environmental stress.

The compositions described herein can be produced by known processes, for example, as a formulation of one or more of the compounds described herein. In some embodiments, the compounds may be optionally mixed with further active ingredients, additives and/or customary formulation auxiliaries, which are then applied in a customary manner, such as, diluted with water, or as what are called tank-mixes by co-dilution of the separately formulated or partially separately formulated component(s) with water. In other embodiments, the compositions are formed in a dry mixture that is added to the soil in, on and/or around the plant. Such formulations may dissolve when exposed to water and thereby be taken up by the roots of the plant. Likewise possible is the application at different times (split application) of the separately formulated or partially separately formulated one or more compounds (e.g., the application of an apocartenoid followed by an additional component, e.g., herbicide, fungicides, etc.). It is also possible, for example, to apply one or more compounds in several portions (sequential application), for example pre-emergence applications followed by post-emergence applications, or early post-emergence applications followed by post-emergence applications at an intermediate or late stage.

In the methods of the disclosure, the composition disclosed herein is contacted with the plant. Any part of the plant may be contacted with the compositions of the disclosure, including tubers, roots, stems, leaves, flowers, and fruits. The composition may be applied directly to seeds or plants, or may be placed in soil in the vicinity of a seed or plant prior to or at the time of planting. In one embodiment, the composition is sprayed on seeds, tubers, or foliage. Seedlings, as well as more mature plants, may be treated. Flowers and fruits may also be treated by spraying. Roots of transplants may be sprayed or dipped in the composition prior to planting. In certain embodiments, the composition of the disclosure may applied, for example, by contacting the unwanted plants (for example harmful plants such as mono- or dicotyledonous weeds or unwanted crop plants), the seed (for example grains, seeds or vegetative propagation organs such as tubers or budded parts of shoots), or the area on which the plants grow (for example the area under cultivation).

The composition of the disclosure, and any additional compounds, can be deployed together (for example as a ready-made formulation or by the tank-mix method) or successively in any sequence, for example by application by irrigating, spraying, watering and sprinkling, or by granule scattering, or by soil injection.

According to their properties, the compositions of the disclosure may be used for pretreatment of the seed of the crop plant (for example for dressing of the seed), or introduced into the seed furrows prior to sowing, or employed alone or together with an additional compound(s) prior to or after emergence of the plants. Pre-emergence treatment includes both the treatment of the area under cultivation (including any water present in the area under cultivation, for example in the case of applications to rice) prior to sowing and the treatment of the area under cultivation in which seeds have been sown but which is not yet covered by growing plants.

In other embodiments, the composition of the disclosure may be applied directly to the plant. In other embodiments, the compositions of the disclosure and any additional compounds are applied directly to the roots of the plant. In yet other embodiments, the compositions of the disclosure and any additional compounds are applied directly to soil around the plant (for example, by soil injection). In yet other embodiments, the compositions of the disclosure and any additional compounds are dissolved in water and applied directly to the plant and/or soil of the plant.

In certain embodiments, contacting the composition to the plant is accomplished, for example, by irrigation, watering, sprinkling, spraying, or broadcasting.

For application, the formulations in commercial form are, if appropriate, diluted in a customary manner, for example in the case of wettable powders, emulsifiable concentrates, dispersions and water-dispersible granules with water. Dust-type formulations, granules for soil application or granules for broadcasting, and sprayable solutions are not normally diluted further with other inert substances prior to application.

The composition of the disclosure may be applied to monocot or dicot plants, and to legumes and non-legumes. In one embodiment, the composition is applied to field-grown plants. In another embodiment, the composition is applied to greenhouse-grown plants. In one embodiment, for example, the composition may be applied to tomatoes, rice, corn, cotton, canola, wheat, barley, sugar beet, turf grass, soybeans, peas, chickpeas, dry beans, peanuts, clover, or alfalfa.

One of skill in the art will recognize that the amounts of the composition (and any additional compounds) which are optimal in each case depend on the nature of the apocarotenoid used and any additional compounds used and on the nature and development stage of the plant stock to be treated, and can be determined in each individual case by simple, routine preliminary tests. In one embodiment, the effective amount of one or more apocarotenoids is at least about 100 nM per m³ of soil, or at least about 10 mg per m³ of soil.

Certain aspects of the disclosure are now explained further via the following nonlimiting examples.

### EXAMPLES

Note that only β-cyclocitral, safranal, dimethyl-β-cyclocitral, and pseudoionone are as such according to one or more aspects of the claimed invention, as set out in the appended claims.

### Materials and Methods

### Plant Growth and Treatment Conditions:

*Arabidopsis thaliana:* All seeds were in the Columbia-0 background. Seeds were sterilized with chlorine gas, incubated in 4 °C water for 2 days, and plated on 1% (wt/vol) Murashige and Skoog (1% MS) media with 1% agar. They were exposed to 100-130 µmol/(m²s¹) illumination and grown vertically under long-day conditions at 22 °C. Plants were analyzed at 7 days after stratification (das), unless noted otherwise. D15-treated media was made using D15 (N-(4-fluorobenzyl)-methoxycinnamic hydroxamic acid) synthesized by LeadGen Labs, LLC solubilized in DMSO. 100 mM D15 stock solution was diluted directly into 1% MS media for final concentrations ranging from 1 µM to 100 µM. For initial experiments testing the ability of apocarotenoids to rescue D15, plants were seeded on 1% MS media containing 30 µM D15 and 100 nM apocarotenoid (diluted from 1 mM stock solutions dissolved in DMSO). For subsequent experiments characterizing the effect of β-cyclocitral, plants were exposed to volatile analytical grade β-cyclocitral (≥ 97%, Sigma Aldrich) using 100 x 15 mm partitioned petri dishes. One side of the partition was filled with 25 µM β-cyclocitral diluted in 1% MS media. Seeds were plated on the other side of the partition, on 1% MS media. This media did not contact the β-cyclocitral, so seedlings were exposed to only volatile β-cyclocitral. For norflurazone treatments, norflurazone (Chem Service) dissolved in DMSO was diluted to a final concentration of 500 nM norflurazone in 1% MS media. For all experiments, including those performed on tomatoes and rice, plants were never exposed to DMSO concentrations that exceeded 0.1%.

*Tomato*: Heinz tomatoes were used for all tomato experiments. Seeds were sterilized using 40% liquid bleach for 30 min, then plated on 1% MS media in 120 x 120 x 17 mm square petri dishes. Seeds were kept in the dark at room temperature for 3 days, and then exposed to 100-130 µmol/(m²s¹) illumination and grown vertically under long-day conditions at 22 °C. For treating tomatoes, β-cyclocitral, norflurazone, and D15 were added directly to the media, at the concentrations described in the figures. Norflurazone was diluted to a final concentration of 1 µM in media. Tomato phenotypes were analyzed at 6 das.

*Rice*: Azucena (*tropical japonica*) seeds were dehulled and germinated in the dark for 3 days at 30 °C. Germinated seeds were transferred to Yoshida's nutrient solution, solidified with 0.25% gelzan (Caisson Laboratories, Inc.), in 2 L glass cylinders, as described previously (Topp, C.N., et al., Proceedings of the National Academy of Sciences of the United States of America, 2013. 110(18): p. E1695-E1704.). Rice roots were imaged at 12 das.

### Root Phenotyping:

To measure lateral root capacity, the primary root apical meristem of each seedling was sterilely excised at 7 days. The plants were given 3 days to grow out lateral roots, and then each emerged lateral root was counted using a dissection microscope, as described previously (Van Norman et al., Proceedings of the National Academy of Sciences of the United States of America, 2014. 111(13): p. E1300-E1309). Luciferase activity was measured as previously described, using exposure times of 7 min (Moreno-Risueno, M.A., et al., Science, 2010. 329(5997): p. 1306-1311). WOX5⁺ and EN7⁺ primordia were counted using a stereo zoom microscope (Zeiss). Lateral and primary root lengths for *Arabidopsis thaliana* and tomato were measured using ImageJ (National Institutes of Health) software. 3D images of rice root architecture were captured using a 360° revolving platform and these images were analyzed using GiA Roots (www.giaroots.org), as described previously by Topp

(2013). Primary root lengths were determined by dissecting the root and measuring the length with a ruler. Shoot mass was measured after removing the shoots and drying them for 10 days at 60 °C.

### Example 1. Role of the carotenoid pathway in regulating lateral root development

First, the effect of D15 on lateral root branching was characterized and is shown Figure 1. D15 is an inhibitor of carotenoid cleavage dioxygenases (CCDs) that preferentially targets LeCCD1 as compared to 9-cis-epoxycarotenoid dioxygenase (LeNCED1) *in vitro* (Van Norman, J.M., et al., PNAS, 2014. 111(13): p. E1300-E1309). NCEDs are a sub-group of CCDs that cleave carotenoids at the 11, 12' positions. The enhanced specificity of D15 towards CCD1 suggests that it preferentially inhibits 7, 8' or 9, 10' cleavages. The maximum effect of D15, at 100 µM, completely inhibited lateral root capacity. By titrating D15 to its IC₅₀ concentration (30 µM), the lateral root capacity was measured with enhanced sensitivity (Figure 1A-B). To determine how D15 inhibits lateral root branching, its effect on the lateral root clock, which establishes the sites of lateral root initiation, was examined. To test this, a synthetic promoter driving luciferase (pDR5:LUC) was utilized. In control roots, luminescence intensity oscillates every six hours (Figure 1C). Only the root cells that experience the peak oscillation intensity become competent to form lateral root pre-branch sites. In D15-treated roots, the peak oscillation intensity was significantly lower as compared to control roots (Figure 1D-E). As expected, D15-treated roots also had significantly decreased numbers of pre-branch sites (p = 0.009). These results suggest that D15 negatively regulates the lateral root clock and inhibits the initiation of lateral root primordia. To further characterize the effect of D15 on lateral root initiation, its effect on two stages of primordia development was determined, as marked by WOX5 (pWOX5:GFP) and EN7 (pEN7:GAL4; UAS:H2A-GFP). These lines allow visualization of the QC at stage 1 of primordia development and the endodermis at stage 3 of development, respectively. The IC₅₀ concentration of D15 significantly decreased the number of stage 1 and stage 3 lateral root primordia by approximately 50% (Figure 1F). This further supports the lateral root clock evidence that D15 inhibits the initiation of lateral root primordia.

D15 did not affect lateral root development after primordia initiation, as measured by comparing its effect on WOX5 and EN7 positive primordia. Additionally, it did not inhibit the emergence of lateral root primordia, as compared to control roots (Figure 1G). This contrasted with the effect of norflurazone, a carotenoid biosynthesis inhibitor, which blocked primordia initiation as well as maintenance, post-initiation development, and emergence (Figure 2). This suggests that while the carotenoid pathway may have roles in numerous stages of lateral root development, D15 is inhibiting an apocarotenoid that plays a specific role in lateral root initiation.

### Example 2. Role of apocarotenoids in regulating lateral root development

Endogenous apocarotenoids were screened for their ability to rescue D15 inhibition of lateral root branching. Most apocarotenoids tested, including ABA and GR24 (a synthetic strigolactone analogue), further decreased lateral root capacity when combined with D15 (Figure 3A). Two apocarotenoids, DHAD and β-cyclocitral, were found to increase lateral root branching in the presence of D15 (Figure 3B). These compounds were previously identified as signaling metabolites that increased plant tolerance to high light stress. These compounds were endogenously present in *Arabidopsis* by GC/MS. To test whether DHAD or β-cyclocitral could fully rescue lateral root branching in D15 treated plants, their concentration was varied to find their maximum capacity for rescue. Even the most effective concentration of DHAD could only partially rescue D15 inhibition. However, nanomolar application of β-cyclocitral could fully rescue D15 inhibition (Figure 4 A-B). This indicates that β-cyclocitral is a novel regulator of lateral root growth and development. Its ability to induce growth at nanomolar concentrations is equivalent to the concentrations at which ABA and strigolactones are active. In addition, apocarotenoids with nearly identical chemical structures, such as dimethyl-β-cyclocitral and β-ionone, did not have the capacity to rescue D15 treatment (Figure 3 A-B). These results suggest that β-cyclocitral, and not an impurity or downstream derivative, is the active signaling molecule regulating lateral root branching.

In addition, at certain concentrations, a combination of β-cyclocitral and DHAD increased lateral root production and shoot mass in plants not subject to D15 inhibition (Figure 3C). These results demonstrate that at low concentrations, β-cyclocitral and DHAD act to induce plant growth. In addition, the combination of β-cyclocitral and DHAD can induce growth compared to either compound alone.

### Example 3. Role of β-cyclocitral in regulating lateral root development

The effect of β-cyclocitral on QC development in lateral root primordia was examined. Surprisingly, β-cyclocitral did not rescue WOX5⁺ primordia initiation in D15-treated plants (Figure 4C). Additionally, it did not increase WOX5⁺ sites in the absence of D15. These results suggested that β-cyclocitral does not have a role in lateral root initiation, and that its rescue of D15 inhibition of lateral root branching may have been a coincidence. Instead, we found that β-cyclocitral did increase the number of EN7⁺ primordia formation in the presence (p = 0.02) and absence (p = 0.12) of D15. β-Cyclocitral also increases lateral root length, with or without co-treatment of D15. This suggests that β-cyclocitral increases lateral root growth and development after stage 1 primordia development. To determine if β-cyclocitral has a more general role in root growth and development, we characterized its effect on primary and lateral root growth. Exogenous volatile application of β-cyclocitral increased primary and lateral root emergence both in the presence and absence of D15 treatment (Figure 6). These results suggest that although β-cyclocitral does not appear to be involved in lateral root initiation, it has a broader role in promoting root growth.

It is difficult to specifically inhibit β-cyclocitral production in plants because there are nine carotenoid cleavage dioxides (CCDs), which are responsible for producing apocarotenoids that appear to be functionally redundant. Due to the challenge of further characterizing the role of β-cyclocitral through genetics, it was determined that β-cyclocitral's effect on root growth was conserved across plant species. Tomatoes were treated with β-cyclocitral, and a significant increase in lateral root length in a dose-dependent manner was found (Figure 5A-B). Norflurazone decreased primary and lateral root growth in tomatoes (Figure 5 A, C). Primary root growth could be fully rescued by application of β-cyclocitral, which also significantly increased primary root length in uninhibited plants. D15 did not have a negative effect on lateral root branching in tomatoes (p = .99), suggesting that its effect on lateral root initiation is not conserved in tomatoes. D15 decreased primary root (but not lateral root) length in tomatoes, but primary root inhibition could not be rescued with exogenous β-cyclocitral (Figure 7). These results suggest that D15 has a different function in tomatoes, either because its enzyme specificity is different, or because CCD-produced apocarotenoids have different roles in tomatoes. β-cyclocitral, however, did induce primary and lateral root growth in both tomato and *Arabidopsis,* suggesting that is a conserved root growth promoter in dicots.

To determine if β-cyclocitral has an effect on root growth in monocots, β-cyclocitral was applied exogenously to rice plants, and changes were measured in root architecture. β-cyclocitral has a striking role in modifying root growth and architecture in *Azucena, a* japonica cultivar (Figure 8). β-cyclocitral-treated root systems were deeper and more compact than control plants (Figure 8B). β-cyclocitral enhanced rice root system depth by dramatically increasing both primary root growth (Figure 8 A, C) and crown root growth (Figure 8 A, E). β-cyclocitral decreased lateral root length in rice (Figure 8D). The overall enhanced root growth caused by β-cyclocitral did not come at a cost to shoot growth, which remained unchanged upon treatment (Figure 9). These results suggest that β-cyclocitral is a conserved root signaling hormone across a variety of plant species. Increased root system depth has been shown to enhance drought resistance in rice, which indicates that exogenous application of β-cyclocitral would be a promising tool for enhancing plant performance during drought.

β-Cyclocitral enhances primary root growth in a conserved manner across species. It additionally promotes lateral root growth in *Arabidopsis* and tomato, and crown root growth in rice. Exogenous modification of these traits in varying environmental conditions can help plants survive different types of stress. In particular, the dramatic ability of β-cyclocitral to generate deeper and more compact rice root systems can enhance drought tolerance. Optionally, β-cyclocitral improves plant performance, especially in harsh environmental conditions.

### Example 4. Role of high concentrations of β-cyclocitral or DHAD

At high concentrations (10 mM) of β-cyclocitral or DHAD, root length, shoot mass, and lateral root growth were inhibited. 10 mM β-cyclocitral, applied only to the root tip, was sufficient to reduce root growth (Figure 10). We examined other apocarotenoids, including safranal, pseudoionone, and dimethyl-β-cyclocitral, and found that they also had herbicidal effects when sprayed on plants (Figure 11A). Interestingly, these compounds appeared to have different methods of lethality. Safranal, for example, changed root cell morphology, whereas pseudoionone caused plants to become chlorotic (Figure 11B-E). These results suggest that β-ionone can negatively regulate plant growth at high concentrations. Optionally, safranal may be detrimental to *Arabidopsis* at concentrations as low as 500 µM.

It is understood that the examples described herein are for illustrative purposes only.

## Claims

1. A method for improving growth in a plant, the method comprising exogenously contacting a composition comprising an effective amount of β-cyclocitral to the plant, a plant part, or a plant seed, wherein the improving growth comprises improving one or more of primary root growth, lateral root growth, crown root growth, and lateral root branching.

2. The method of claim 1, wherein the growth of the plant is improved by at least about 10% compared to the plant not contacted with the composition.

3. A method for improving drought tolerance in a plant, the method comprising exogenously contacting a composition comprising an effective amount of β-cyclocitral to the plant, a plant part, or a plant seed.

4. A method for reducing growth in a plant, the method comprising exogenously contacting a composition comprising an effective amount of β-cyclocitral, safranal, dimethyl-β-cyclocitral, or pseudoionone, or a combination thereof, to the plant, a plant part, or a plant seed.

5. The method of claim 4, wherein the growth of the plant is reduced by at least about 20% compared to the plant not contacted with the composition.

6. The method of any of claims 1 - 5, wherein contacting is by irrigating the plant.

7. The method according to any of claims 1 - 6, wherein the composition further comprises one or more fertilizers (e.g., calcium, nitrogen, potassium, phosphorous), micronutrients (e.g., metal ions), insecticides, fungicides, nematocides, bactericides, acaricides, herbicides, plant nutrients, rooting stimulants, chemosterilants, semiochemicals, repellents, attractants, pheromories, feeding stimulants, microbial inocula, or entomopathogenic bacteria, viruses, or fungi.

8. The method according to any of claims 1 - 7, wherein the composition further comprises an agriculturally acceptable carrier, excipient, and/or diluent.

## Patentansprüche

1. Verfahren zur Verbesserung des Wachstums einer Pflanze, wobei das Verfahren das exogene Inkontaktbringen einer Zusammensetzung, die eine wirksame Menge an β-Cyclocitral umfasst, mit der Pflanze, einem Pflanzenteil oder einem Pflanzensamen umfasst, wobei die Verbesserung des Wachstums die Verbesserung eines oder mehrerer von Primärwurzelwachstum, Lateralwurzelwachstum, Kronenwurzelwachstum und Lateralwurzelverzweigung umfasst.

2. Verfahren nach Anspruch 1, wobei das Wachstum der Pflanze um mindestens etwa 10 % im Vergleich zu der nicht mit der Zusammensetzung in Kontakt gebrachten Pflanze verbessert wird.

3. Verfahren zur Verbesserung der Trockenheitstoleranz einer Pflanze, wobei das Verfahren das exogene Inkontaktbringen einer Zusammensetzung, die eine wirksame Menge an β-Cyclocitral umfasst, mit der Pflanze, einem Pflanzenteil oder einem Pflanzensamen umfasst.

4. Verfahren zur Verringerung des Wachstums einer Pflanze, wobei das Verfahren das exogene Inkontaktbringen einer Zusammensetzung, die eine wirksame Menge an β-Cyclocitral, Safranal, Dimethyl-β-cyclocitral oder Pseudoionon oder einer Kombination davon umfasst, mit der Pflanze, einem Pflanzenteil oder einem Pflanzensamen umfasst.

5. Verfahren nach Anspruch 4, wobei das Wachstum der Pflanze um mindestens etwa 20 % im Vergleich zu der nicht mit der Zusammensetzung in Kontakt gebrachten Pflanze verringert wird.

6. Verfahren nach einem der Ansprüche 1 - 5, wobei das Inkontaktbringen durch Bewässerung der Pflanze erfolgt.

7. Verfahren nach einem der Ansprüche 1 - 6, wobei die Zusammensetzung ferner eines oder mehrere von Düngemitteln (z. B. Calcium, Stickstoff, Kalium, Phosphor), Mikronährstoffen (z. B. Metallionen), Insektiziden, Fungiziden, Nematiziden, Bakteriziden, Akariziden, Herbiziden, Pflanzennährstoffen, Bewurzelungsstimulanzien, Chemosterilantien, Semiochemikalien, Repellentien, Lockstoffen, Pheromonen, Fraßstimulanzien, mikrobiellen Inokula oder entomopathogenen Bakterien, Viren oder Pilzen umfasst.

8. Verfahren nach einem der Ansprüche 1 - 7, wobei die Zusammensetzung ferner einen landwirtschaftlich akzeptablen Träger, Hilfsstoff und/oder Verdünnungsmittel umfasst.

## Revendications

1. Méthode pour améliorer la croissance d'une plante, la méthode comprenant la mise en contact exogène d'une composition comprenant une quantité efficace de β-cyclocitral avec la plante, une partie de plante ou une graine de plante, dans laquelle l'amélioration de la croissance comprend l'amélioration d'une ou plusieurs parmi la croissance des racines primaires, la croissance des racines latérales, la croissance des racines coronaires et la ramification des racines latérales.

2. Méthode selon la revendication 1, dans laquelle la croissance de la plante est améliorée d'au moins environ 10 % par rapport à la plante non mise en contact avec la composition.

3. Méthode pour améliorer la tolérance à la sécheresse d'une plante, la méthode comprenant la mise en contact exogène d'une composition comprenant une quantité efficace de β-cyclocitral avec la plante, une partie de plante ou une graine de plante.

4. Méthode pour réduire la croissance d'une plante, la méthode comprenant la mise en contact exogène d'une composition comprenant une quantité efficace de β-cyclocitral, de safranal, de diméthyl-β-cyclocitral, ou de pseudoïonone, ou d'une combinaison de ceux-ci, avec la plante, une partie de plante ou une graine de plante.

5. Méthode selon la revendication 4, dans laquelle la croissance de la plante est réduite d'au moins environ 20 % par rapport à la plante non mise en contact avec la composition.

6. Méthode selon l'une quelconque des revendications 1 - 5, dans laquelle la mise en contact est réalisée par irrigation de la plante.

7. Méthode selon l'une quelconque des revendications 1 - 6, dans laquelle la composition comprend en outre un ou plusieurs fertilisants (par exemple, calcium, azote, potassium, phosphore), micronutriments (par exemple, ions métalliques), insecticides, fongicides, nématocides, bactéricides, acaricides, herbicides, nutriments pour plantes, stimulants d'enracinement, chimiostérilisants, sémiochimiques, répulsifs, attractants, phéromones, stimulants d'alimentation, inocula microbiens, ou bactéries, virus ou champignons entomopathogènes.

8. Méthode selon l'une quelconque des revendications 1 - 7, dans laquelle la composition comprend en outre un support, excipient et/ou diluant acceptable sur le plan agricole.
